# EUROPÄISCHE PATENTSCHRIFT

(11) **EP 2 819 615 B1**
(45) Veröffentlichungstag und Bekanntmachung des Hinweises auf die Patenterteilung: **03.01.2018**
(21) Anmeldenummer: 13702397.4
(22) Anmeldetag: 24.01.2013
(51) Int. Cl.: A61C 17/06, A61M 1/00

(54) **SAUGMASCHINE MIT GEHÄUSE AUS EXPANDIERTEM KUNSTSTOFF**
SUCTION MACHINE COMPRISING A HOUSING MADE OF EXPANDED PLASTIC MATERIAL
MACHINE D'ASPIRATION POURVUE D'UN BOÎTIER EN PLASTIQUE EXPANSÉ

(30) Priorität: 29.02.2012 DE 102012101642
(43) Veröffentlichungstag der Anmeldung: 07.01.2015
(73) Patentinhaber: Dürr Dental AG, 74321 Bietigheim-Bissingen (DE)
(72) Erfinder: MAUTHE, Ralf, 74348 Lauffen (DE)
(74) Vertreter: Ostertag & Partner Patentanwälte mbB
(86) Internationale Anmeldenummer: PCT/EP2013/051265
(87) Internationale Veröffentlichungsnummer: WO 2013/127570

(56) Entgegenhaltungen:
- EP-A1- 0 263 344
- US-A- 3 078 579
- US-A1- 2007 179 460

## Beschreibung

Die vorliegende Erfindung betrifft eine Saugvorrichtung für dentale, medizinische oder industrielle Zwecke mit einer Sauganlage und einem Gehäuse, in das die Sauganlage aufgenommen ist.

Saugvorrichtungen bzw. Saugsysteme sind allgemein bekannt und werden beispielsweise von der Anmelderin in unterschiedlichsten Varianten angeboten. So ist beispielsweise eine Saugvorrichtung aus der Druckschrift DE 10 2006 058 955 A1 bekannt.

Aus der Druckschrift US 3,078,579 ist eine Saugvorrichtung für dentale Zwecke bekannt, die ein aus mehreren Teilen bestehendes Gehäuse besitzt, das mit geräuschisolierendem Material wie Steinwolle oder Fiberglass ausgekleidet ist.

Aus der Druckschrift US 5,323,562 ist eine Arbeitsstation für Zahntechniker bekannt, bei welcher in einem Staubschutzgehäuse beispielsweise Zahnfüllungen bearbeitet werden. Die Arbeitsstation umfasst eine Absaugvorrichtung und hat ein Gehäuse aus geschäumtem Material.

Grundsätzlich dienen solche Saugsysteme beispielsweise dazu, die bei einer zahnmedizinischen Behandlung entstehenden Stoffe, wie Abwasser, Blut, Speichel, Dentin oder unterschiedliche Füllungswerkstoffe, aus der Mundhöhle des Patienten abzusaugen und optional zu separieren und abzuscheiden. Ein solches Saugsystem für dentale Zwecke umfasst mehrere Komponenten, wie beispielsweise Saugeinheit, Separiereinheit, Abscheidereinheit, Steuerungseinheit etc., die miteinander zu einem Gesamtsystem verbunden werden müssen. Einzelne Komponenten werden beispielsweise auf Blechbiegeteile aufgestellt oder stehen auf einem Gehäuseteil, wie beispielsweise einem Schalldämpfergehäuse. Bei der Auslieferung sind die Saugsysteme nicht direkt anschlussfertig, insbesondere weil auch die Steuerung in einem Schaltkasten extra untergebracht ist und zunächst installiert werden muss. Um die im Betrieb eines solchen Saugsystems entstehenden Geräusche zu minimieren, werden zusätzliche geräuschmindernde Maßnahmen erforderlich.

Obgleich sich solche Saugsysteme in der Praxis bewährt haben, bleibt weiterhin der Wunsch bestehen, Saugsysteme bzw. Saugvorrichtungen kompakter auszugestalten, den Aufbau zu vereinfachen und die Geräuschemission zu reduzieren.

Diese Aufgabe wird durch eine Saugvorrichtung nach Anspruch 1 gelöst.

Das heißt mit anderen Worten, dass die Saugvorrichtung nunmehr über ein Gehäuse verfügt, das nicht nur einfach die einzelnen Komponenten der Saugvorrichtung umgibt sondern vielmehr noch zusätzliche Funktionen bereitstellt, nämlich insbesondere funktionale Komponenten selbst ausbildet. In dem aus zumindest zwei zusammensetzbaren Gehäuseschalen gebildeten Gehäuse sind folglich funktionale Komponenten integriert. Zusätzlich ist in zumindest einer der Gehäuseschalen zumindest eine Abstützfläche ausgeformt, das heißt integriert, die eine Komponente der Sauganlage abstützt und/oder hält, so dass auf spezielle Rahmenelemente, Bleche, etc. verzichtet werden kann. Infolgedessen werden auch keine bzw. nur wenige Befestigungsmittel, wie Schrauben etc., erforderlich, da die einzelnen Komponenten in darin ausgebildeten Ausformungen aufgenommen und gehalten werden.

Die Vorteile einer solchen Saugvorrichtung sind vielfältig, wobei hier insbesondere ein kompakter Aufbau mit geringem Aufbau-Aufwand zu nennen ist. Ein weiterer wichtiger Vorteil ist darin zu sehen, dass durch Verwendung eines geschäumten Materials für die Gehäuseschalen eine sehr hohe Geräuschdämmung erzielbar ist. Noch ein weiterer Vorteil ist darin zu sehen, dass durch Integration funktionaler Komponenten in den Gehäuseschalen der bauliche Aufwand reduziert wird, da diese funktionalen Komponenten nicht separat vorgesehen werden müssen. Die Anzahl der erforderlichen Funktionsteile kann damit reduziert werden, was große Auswirkungen nicht nur auf die Kosten sondern auch auf den Montageaufwand hat.

Dem Benutzer kann mit diesen Maßnahmen eine Saugvorrichtung bereitgestellt werden, die mit minimalem Installationsaufwand in Betrieb genommen werden kann.

Bei einer bevorzugten Weiterbildung umfasst die Sauganlage zumindest eine Saugeinheit und/oder zumindest eine Separiereinheit, die die abgesaugte Luft von den flüssigen bzw. festen Stoffen trennt, und/oder eine Abscheidereinheit, die bspw. Amalgam abscheidet.

Bei einer bevorzugten Weiterbildung handelt es sich bei dem schäumbaren Material um einen Kunststoff. Insbesondere werden die Gehäuseschalen aus einem Kunststoff expandiert.

Ein solcher schäumbarer Kunststoff ist beispielsweise Polypropylen. Selbstverständlich können neben expandierbaren Kunststoffen auch Naturmaterialien, wie beispielsweise Maisschaum, verwendet werden.

Wie zuvor bereits ausgeführt, ist ein wesentlicher Aspekt der Erfindung darin zu sehen, dass zumindest eine der Gehäuseschalen zumindest eine funktionale Komponente der Sauganlage ausbildet. Bei der funktionalen Komponente kann es sich beispielsweise um einen Luftführungskanal, einen oder mehrere Standfüße, ein Schalldämmungselement, ein Schwingungsentkoppelungselement und/oder ein Halteelement für Leitungen, Anschlüsse etc. handeln.

Das bedeutet mit anderen Worten, dass in einer oder beiden geschäumten Gehäuseschalen beispielsweise Kanäle für die Luftführung vorgesehen sind, so dass auf die bisherigen Schlauchleitungen verzichtet werden kann. Ferner können in den geschäumten Gehäuseschalen beispielsweise Klemmbereiche vorgesehen werden, in die dann Leitungen etc. eingesteckt und festgehalten werden können. Schließlich wirken die Gehäuseschalen schalldämmend und schwingungsentkoppelnd, wobei diese Effekte durch entsprechende Wahl der Innengeometrie weiter verbessert werden können. Aufgrund der hohen Gestaltungsflexibilität der Gehäuseschalen ist es denkbar, weitere funktionale Komponenten in diese zu integrieren.

In zumindest einer der Gehäuseschalen ist zumindest ein Aufnahmebereich ausgeformt, der zur Aufnahme von zumindest einer Saugvorrichtungs-Komponente dient. Eine solche Saugvorrichtungs-Komponente ist beispielsweise ein elektrisches Anschlusselement, ein Schlauchanschlusselement, ein Steuerungsgehäuse, eine elektrische Leitung oder eine Kabelführung für Leitungen.

Das heißt mit anderen Worten, dass in den Gehäuseschalen Ausnehmungen vorgesehen sind, in die die Saugvorrichtungs-Komponenten eingebracht werden können und die die eingebrachte Komponente dann zumindest teilweise umgeben. So lassen sich innerhalb der beiden Gehäuseschalen bestimmte Komponenten, wie beispielsweise ein Steuerungsgehäuse, von anderen Komponenten abgrenzen bzw. trennen, was beispielsweise Vorteile bei der Luftführung, insbesondere zu Kühlzwecken, hat. Elektrische Leitungen oder andere Leitungen lassen sich in solchen Ausnehmungen in den Gehäuseschalen einfach und definiert führen, so dass es beispielsweise nicht zu einer Kontaktierung mit heißen Teilen innerhalb des Gehäuses kommen kann.

Zusammen mit den in den Gehäuseschalen vorgesehenen Abstützflächen können die Komponenten, wie beispielsweise Steuerungseinheit, Lüftereinheit, Separiereinheit und/oder Antriebseinheit, auf einfache Weise in den Gehäuseschalen aufgenommen werden, da einerseits die Positionen für die einzelnen Komponenten fest vorgegeben sind und andererseits keine weiteren Befestigungsmittel etc. erforderlich werden, da die Komponenten über die Abstützflächen bei zusammengesetzten Gehäuseschalen sicher gehalten werden.

Insgesamt entsteht eine Saugvorrichtung, die leichter und kompakter gebaut werden kann, wobei sich eine Gewichtseinsparung von 30 bis 40 % realisieren lässt. Ferner werden die Geräuschemissionen deutlich reduziert dadurch, dass die jeweiligen Komponenten durch die Gehäuseschalen gekapselt sind. Der Einsatz von Kleinteilen wie Schrauben, Kabelklemmen, Schwingungsdämpfer usw. werden auf ein Minimum reduziert, so dass sich daraus deutliche Vorteile bei der Montage ergeben.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Vorteile und Ausgestaltungen der Erfindung ergeben sich aus der nachfolgenden detaillierten Beschreibung einer bevorzugten Ausführungsform und der beiliegenden Zeichnung. Dabei zeigen:
- Fig. 1: eine erfindungsgemäße Saugvorrichtung in einer ersten perspektivischen Ansicht;
- Fig. 2: die erfindungsgemäße Saugvorrichtung in einer zweiten perspektivischen Ansicht;
- Fig. 3a, b: die erfindungsgemäße Saugvorrichtung in einer perspektivischen Explosionsdarstellung;
- Fig. 4a, b: jeweils eine Innenansicht einer Gehäuseschale der erfindungsgemäßen Saugvorrichtung in perspektivischer Darstellung;
- Fig. 5a, b: die beiden Gehäuseschalen mit eingelegten Einzelkomponenten in perspektivischer Darstellung;
- Fig. 6: eine Schnittansicht einer erfindungsgemäßen Saugvorrichtung zur Erläuterung von funktionalen Komponenten; und
- Fig. 7: eine weitere Schnittansicht der erfindungsgemäßen Saugvorrichtung.

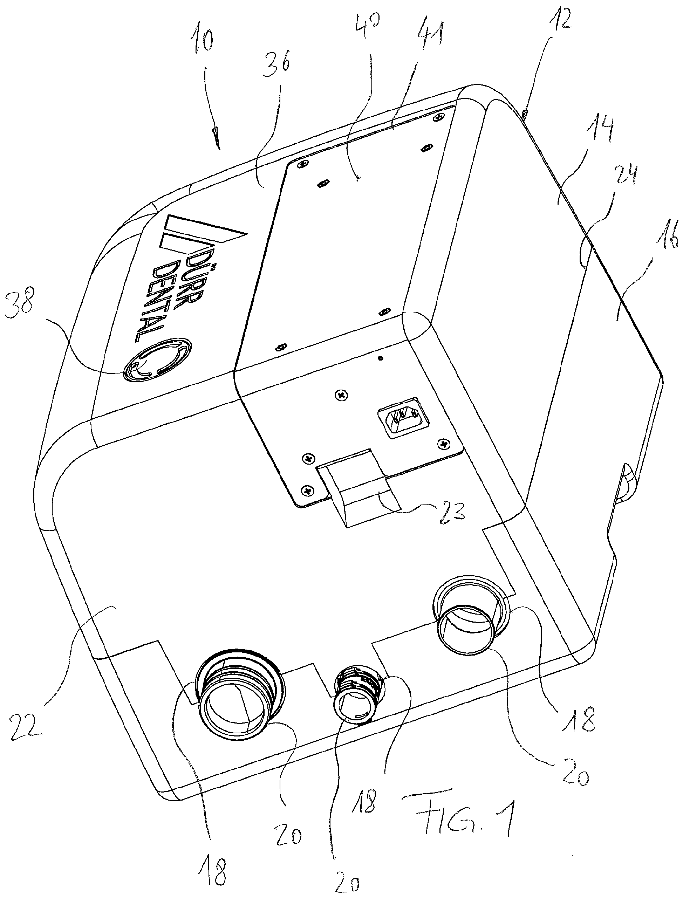

In Fig. 1 ist ein Saugsystem bzw. eine Saugvorrichtung in perspektivischer Darstellung gezeigt und mit dem Bezugszeichen 10 gekennzeichnet. Eine solche Saugvorrichtung wird beispielsweise für dentale Zwecke eingesetzt und dient dazu, Flüssigkeiten, wie Speichel, Blut etc. und feste Stoffe, wie beispielsweise Kunststoff, Keramik, Amalgam, Edelmetalle etc., aus der Mundhöhle eines behandelten Patienten abzusaugen. Die Saugvorrichtung 10 ist dabei entweder in unmittelbarer Nähe des Behandlungsstuhls vorgesehen oder entfernt davon, beispielsweise in einem anderen Raum. Darüber hinaus kann eine solche Saugvorrichtung 10 für einen oder für mehrere Behandlungsplätze ausgelegt sein. Ohne eine solche Absaugung würde sich ein von modernen Schnelllaufinstrumenten eingesetztes Kühlspray als Aerosolwolke im gesamten Behandlungszimmer ausbreiten. Die in einer solchen Aerosolwolke enthaltenen Keime, Bakterien und Viren würden eine Infektionsgefahr entstehen lassen, was verhindert werden soll.

Die Saugvorrichtung 10 weist hierfür verschiedene Baugruppen auf, die in einem Gehäuse 12 untergebracht sind. Dieses Gehäuse 12 besteht aus mehreren, bevorzugt zwei, Gehäuseschalen 14, 16. Die beiden Gehäuseschalen 14, 16 sind zusammensetzbar, was unter anderem bedeutet, dass die einander zugewandten Flächen der beiden Gehäuseschalen zueinanderpassend geformt sind. Insgesamt ergibt sich ein im Wesentlichen geschlossenes Gehäuse 12, das lediglich Öffnungen für die Zu- bzw. Abführung von Energie, Luft und Flüssigkeiten besitzt.

Beide Gehäuseschalen 14, 16 sind bevorzugt aus dem gleichen Material gefertigt, wobei es sich vorzugsweise um einen schäumbaren Kunststoff handelt. Ein solcher schäumbarer Kunststoff ist beispielsweise Polypropylen. Selbstverständlich sind auch andere schäumbare Materialien, insbesondere Naturmaterialien, als Gehäusewerkstoff denkbar. Wie später noch erläutert werden wird, wird das Material für das Gehäuse 12 unter anderem nach seiner Dämpfungsfähigkeit bezüglich Schall und Vibrationen ausgewählt.

Das vorzugsweise quaderförmige Gehäuse 12 besitzt an einer seiner Seiten 22 drei kreisrunde Öffnungen 18, in die Anschlusselemente, beispielsweise für Schlauch- oder Rohrleitungen, eingelegt sind. Die mit dem Bezugszeichen 20 gekennzeichneten Anschlusselemente können Verbindungsflächen am Umfang, beispielsweise Gewinde, aufweisen, so dass Schlauchleitungen einfach angeschlossen werden können. Im vorliegenden Ausführungsbeispiel ist die in Fig. 1 rechte Öffnung 18 für das Ausblasen von Abluft, die Öffnung 18 für das Ableiten von abgeschiedenem Sekret und die linke Öffnung 18 zum Ansaugen von Luft und Sekret aus der Mundhöhle eines Patienten vorgesehen. Im oberen Bereich dieser Seite 22 ist eine weitere schlitzartige Öffnung 23 vorgesehen, die ebenfalls zum Ausblasen von Luft dient.

Wie bereits erwähnt, setzt sich das Gehäuse 12 aus den beiden Gehäuseschalen 14 und 16 zusammen, wobei die Trennlinie der beiden Gehäuseschalen mit dem Bezugszeichen 24 gekennzeichnet ist. Die drei kreisrunden Öffnungen 18 werden von dieser Trennlinie 24 auf der Seite 22 geschnitten, was bedeutet, dass diese Öffnungen 18 im Gehäuse 12 durch randoffene kreissegmentförmige Ausnehmungen in den beiden Gehäuseschalen ausgebildet werden.

An der unteren, den Boden 26 bildenden Seite der unteren Gehäuseschale 16 sind ebenfalls Öffnungen 28 vorgesehen, die eine Verbindung zum Innenraum des Gehäuses 12 schaffen. Auch diese Öffnungen 28 können dazu genutzt werden, Luft einzusaugen oder auszublasen.

Am Boden 26 sind ferner vier Standfüße 30 ausgebildet, wobei die Standfüße 30 integraler Bestandteil der unteren Gehäuseschale 16 sind. Das heißt mit anderen Worten, dass diese Standfüße 30 bei der Herstellung der Gehäuseschale mit ausgeformt werden, und mithin nicht in irgendeiner Weise mit dem Boden, beispielsweise durch Verkleben oder Verschrauben, verbunden werden müssen.

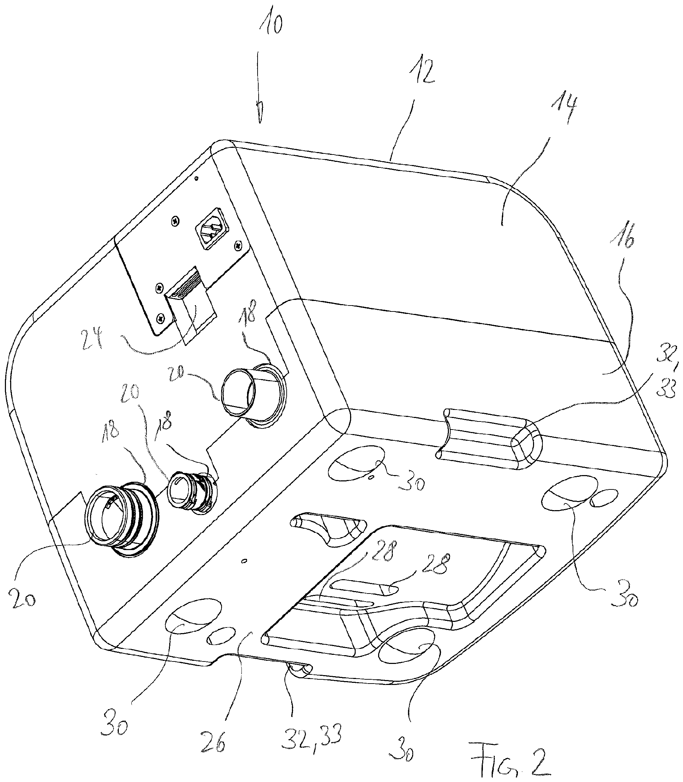

Schließlich lässt die Darstellung in Fig. 2 noch zwei am Übergang zwischen Boden und Seite vorgesehene Ausnehmungen 32 erkennen, die als Griffmulden 33 dienen.

Eine weitere Öffnung im Gehäuse 12 findet sich auf der oberen Seite, dem Deckel 36 der oberen Gehäuseschale 14, und ist mit dem Bezugszeichen 38 gekennzeichnet. Diese Öffnung 38 ist schlitzartig ausgebildet, um zu vermeiden, dass Fremdkörper in das Innere des Gehäuses 12 gelangen können.

Schließlich ist von außen noch ein Elektronikgehäuse 40 zu erkennen, dessen außen liegende Abdeckplatte 41 in etwa L-förmig ist und sich von der Deckelseite auf die Seite 22 erstreckt. Dieses Elektronikgehäuse 40 enthält die für die Steuerung und Energieversorgung erforderlichen Bauteile und wird von außen über einen Netzstecker, der in die zu erkennende Netzbuchse eingesteckt werden kann, versorgt. Die Abdeckplatte 41 endet auf der Seite 22 im Bereich der Öffnung 23 und ist so ausgebildet, dass Luft aus dem Gehäuse 12 durch die Öffnung 23 nach außen strömen kann.

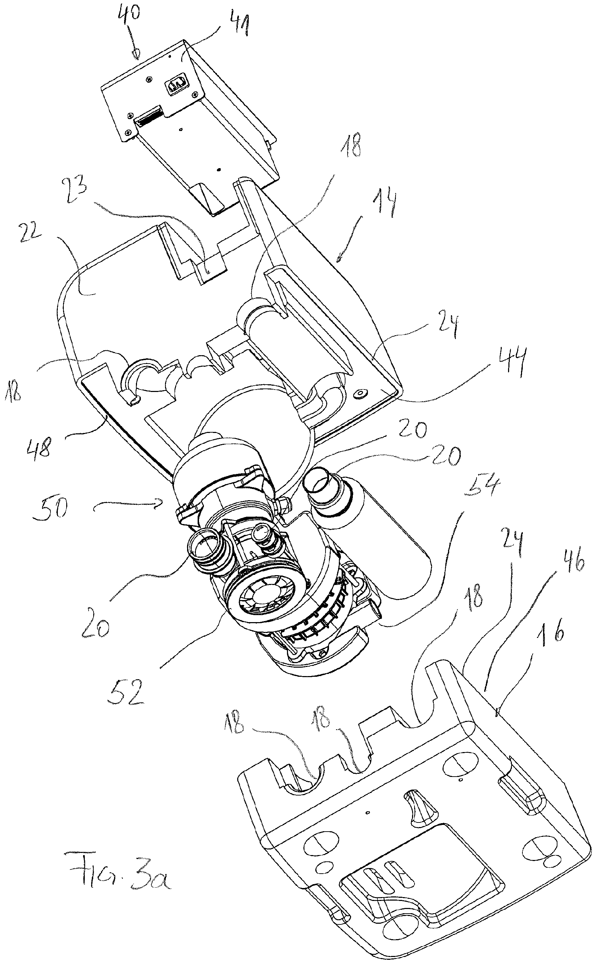

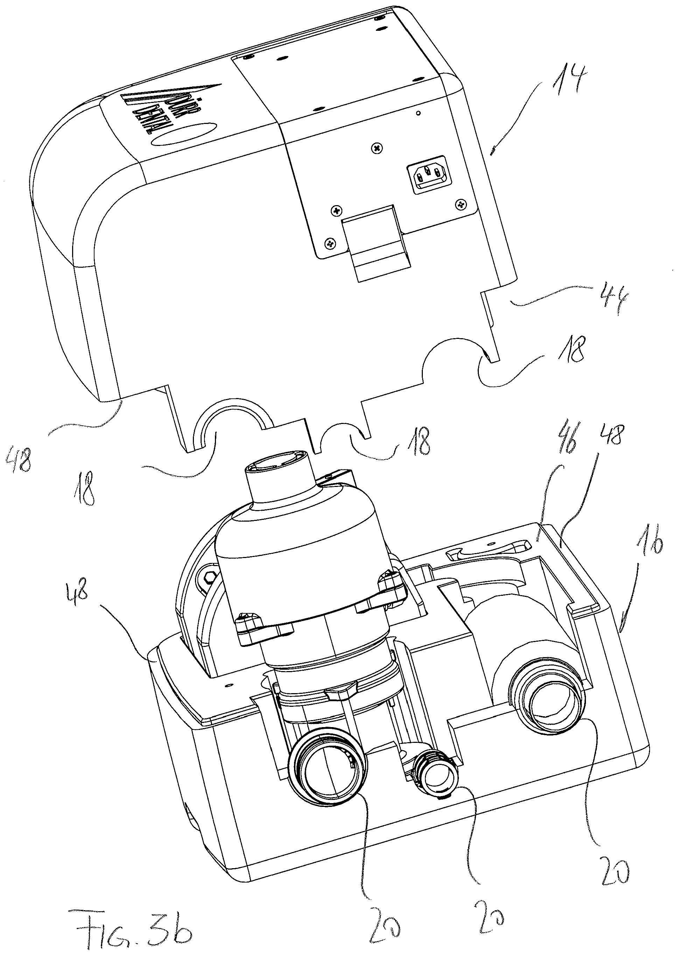

In den Fig. 3a und 3b ist das Gehäuse 12 in geöffneter Position dargestellt, so dass das Innenleben des Gehäuses 12 sichtbar wird. Im Gehäuse 12 ist eine Sauganlage aufgenommen, die allgemein mit dem Bezugszeichen 50 gekennzeichnet ist. Diese Sauganlage 50 kann mehrere Baugruppen aufweisen, nämlich insbesondere eine Saugeinheit 54 bzw. Saugmaschine 54, bspw. eine Separiereinheit 52, die bevorzugt als Radial-Saugmaschine 55 ausgebildet ist, und bspw. zusätzlich eine Abscheidereinheit zum Abscheiden von Amalgam oder anderes Zubehör. Da der Aufbau einer solchen Sauganlage allgemein bekannt ist, soll darauf nicht weiter eingegangen werden. An dieser Stelle sei noch angemerkt, dass die Sauganlage 50 auch mehrere der vorgenannten einzelnen Baugruppen umfassen kann, also bspw. zwei oder mehr Saugmaschinen.

Zur Aufnahme der Sauganlage 50 weisen die beiden Gehäuseschalen 14, 16 an den einander zugewandten Seiten 44 und 46 an die Sauganlage 50 angepasste Ausnehmungen auf, so dass, wie sich aus Fig. 3b ergibt, die einzelnen Baugruppen der Sauganlage 50 in die untere Gehäuseschale 16 einfach eingelegt werden können und durch Aufsetzen der oberen Gehäuseschale 14 dann sicher im Gehäuse 12 gehalten sind. Es ist folglich innerhalb des Gehäuses 12 kein Metallgestell oder Ähnliches erforderlich, um die Baugruppen der Sauganlage 50 in einer festen Position zueinander zu halten.

Die beiden Innenseiten 44, 46 der Gehäuseschalen 14, 16 besitzen jeweils einen Rand 48, der eine Abstufung besitzt. Diese Abstufung ermöglicht einerseits eine definierte Positionierung der oberen Gehäuseschale 14 auf der unteren Gehäuseschale 16, da die Abstufung 48 an der oberen Gehäuseschale 14 ein "Negativ" der unteren Abstufung ist. Ferner führt diese Abstufung zu einer dichteren Verbindung, so dass der Innenraum des Gehäuses 12 besser abgekapselt wird.

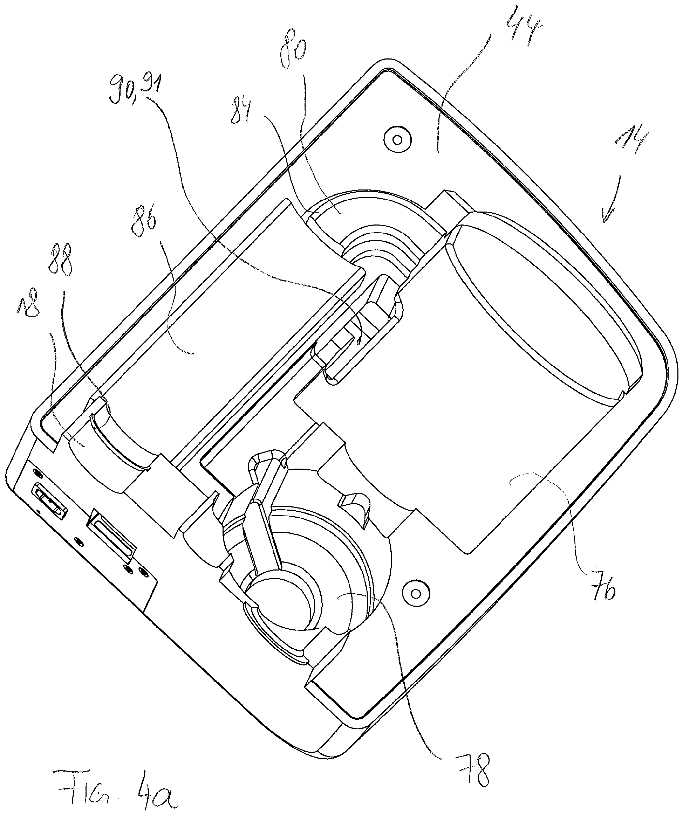

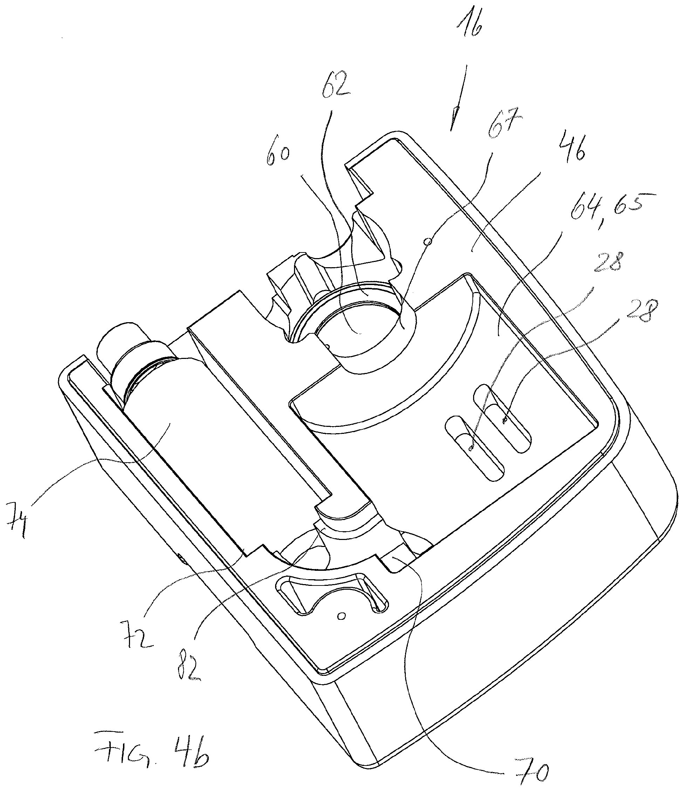

In den beiden Fig. 4a und 4b sind nun die Innenseiten der beiden Gehäuseschalen 14, 16 ohne eingelegte Sauganlage 50 zu sehen. Die untere Gehäuseschale 16 weist eine erste Vertiefung 60 auf, die an den unteren Teil der Separiereinheit 52 angepasst ist und diese abstützt. An diese Vertiefung 60 mit einem ringförmigen Abstützrand 62 schließt sich eine weitere Vertiefung 64 an, die die Form eines liegenden Halbzylinders besitzt. Diese Vertiefung 64 ist an die Saugeinheit 54 angepasst und stützt diese ab. Die Vertiefung 64 hat damit die Funktion einer Abstützfläche für die Saugeinheit, wobei in dieser Vertiefung 64 die beiden Öffnungen 28 vorgesehen sind.

Die beiden Vertiefungen 60, 64 sind durch eine Wand getrennt, die jedoch einen randoffenen halbkreisförmigen Ausschnitt 67 aufweist, der zur Aufnahme eines rohrförmigen Verbindungselements zwischen der Separiereinheit 52 und der Saugeinheit 54 vorgesehen ist.

Weiter weist die Innenseite der unteren Gehäuseschale 16 eine Vertiefung 70 auf, die auf ihrer einen Seite in die Vertiefung 64 und auf ihrer anderen Seite in eine halbzylindrische Ausnehmung bzw. Vertiefung 72 mündet, die ihrerseits in die Öffnung 18 für den Luftauslass mündet. In diese halbzylindrische Vertiefung 72 ist ein rohrförmiges Schalldämpfungselement 74 eingelegt. Die beiden Vertiefungen 70, 72 sind so ausgeführt, dass ein Luftstrom aus dem Vertiefungsbereich 64 durch die Vertiefung 70 und das in der Vertiefung 72 liegende Schalldämpfungselement 74 durch die Öffnung 18 nach außen geführt werden kann.

Auf der Innenseite 44 der oberen Gehäuseschale 14 sind ebenfalls Vertiefungen vorgesehen, die an die entsprechende Geometrie der Sauganlage 50 angepasst sind. So ist eine zylindrische Vertiefung 76 vorgesehen, die zusammen mit der Vertiefung 64 in der unteren Gehäuseschale 16 dann eine im Wesentlichen zylindrische Aufnahme für die Saugeinheit 54 bildet. Ferner ist eine Vertiefung 78 für die Separiereinheit 52 vorgesehen, deren Geometrie an den oberen Teil der Separiereinheit 52 angepasst ist und zusammen mit der Vertiefung 76 die Sauganlage 50 sicher im Gehäuse 12 hält.

In der Gehäuseschale 14 ist ferner ein erhabener Bereich 80 vorgesehen, der an die Form der Vertiefung 70 angepasst ist und bei aufgesetzten Gehäuseschalen einen nach oben geschlossenen Kanal mit der Vertiefung 70 bildet. Dieser erhabene Bereich 80 dichtet sozusagen die Vertiefung 70 nach oben ab. Für diese Abdichtungen sind beispielsweise Aufsatzflächen 82 in den seitlichen Begrenzungen der Vertiefung 70 vorgesehen, die mit entsprechenden Flächen 84 zusammenwirken. Damit wird alleine durch die Ausgestaltung der Innenseiten der beiden Gehäuseschalen 14, 16 ein Luftkanal gebildet, vergleichbar einem Rohrabschnitt.

Um das Schalldämpfungselement 74 sicher in der Vertiefung 72 zu halten, ist eine entsprechend geformte Abstützfläche bzw. Haltefläche 86 in der oberen Gehäuseschale 14 vorgesehen, die sich von dem erhabenen Bereich 80 zu der in Fig. 1 rechts gezeigten Öffnung 18 erstreckt. Innerhalb dieser Öffnung 18 ist in Fig. 4a eine in Umfangsrichtung verlaufende Nut 88 zu erkennen, die beispielsweise zur Aufnahme eines Abdichtrings für ein Anschlusselement 20 dient.

In Fig. 4a ist im Bereich zwischen der Abstützfläche 86 und der Vertiefung 76 eine Öffnung 90 vorgesehen, die in den Bereich des Elektronikgehäuses 40 mündet. Diese Öffnung 90 hat die Funktion eines Kanals 91, der Luft aus dem Bereich der Saugeinheit 54 in den Bereich des Elektronikgehäuses 40 leiten soll.

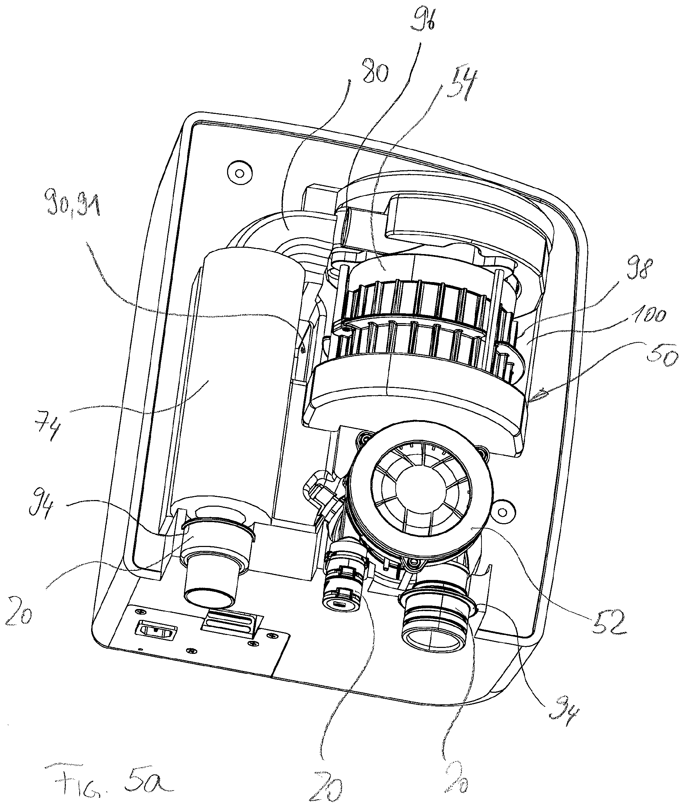

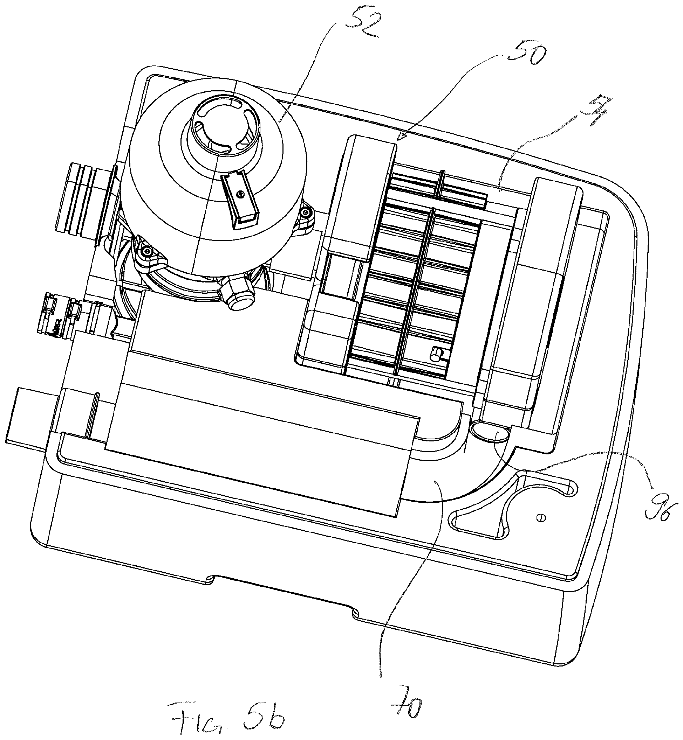

Die beiden Fig. 5a und 5b zeigen die Innenseiten 44, 46 der beiden Gehäuseschalen 14 bzw. 16 mit eingelegter Sauganlage 50, Schalldämpfungselement 74 und Anschlusselementen 20.

Diese Anschlusselemente 20 besitzen bevorzugt einen ringförmigen Steg bzw. Flansch 94, der in die Nut 88 greift und damit die Öffnung 20 einerseits abdichtet und andererseits das Anschlusselement 20 gegen ein Verschieben in axialer Richtung sichert.

Wie sich aus den beiden Fig. 5a, 5b ebenfalls ergibt, verfügt die als Radial-Saugmaschine 55 ausgelegte Saugeinheit 54 über einen seitlichen Luftauslass 96, aus dem die von Sekret befreite Saugluft ausgeblasen wird. Der Luftauslass 96 mündet in die Vertiefung 70, die zusammen mit dem erhabenen Bereich 80 in der oberen Gehäuseschale 14 einen Luftkanal bildet, der in das rohrförmige Schalldämpfungselement 74 mündet. Dieses Schalldämpfungselement 74 führt dann die ausgeblasene Luft zu der Öffnung 18, in der das Anschlusselement 20 vorgesehen ist.

In Fig. 5a ist zu erkennen, dass die Saugeinheit 54 - in Längsrichtung gesehen - einen mittleren Bereich 98 mit Kühlrippen aufweist, der durchmesserkleiner ist als die beiden äußeren Längsbereiche. Da die beiden Vertiefungen 64, 76 in Kombination einen - in Längsrichtung gesehen - gleichen Durchmesser besitzen, entsteht ein freier Raum 100 um diesen mittleren Bereich 98 herum. Dieser freie Raum 100 besitzt dabei eine Verbindung zu der Öffnung 90.

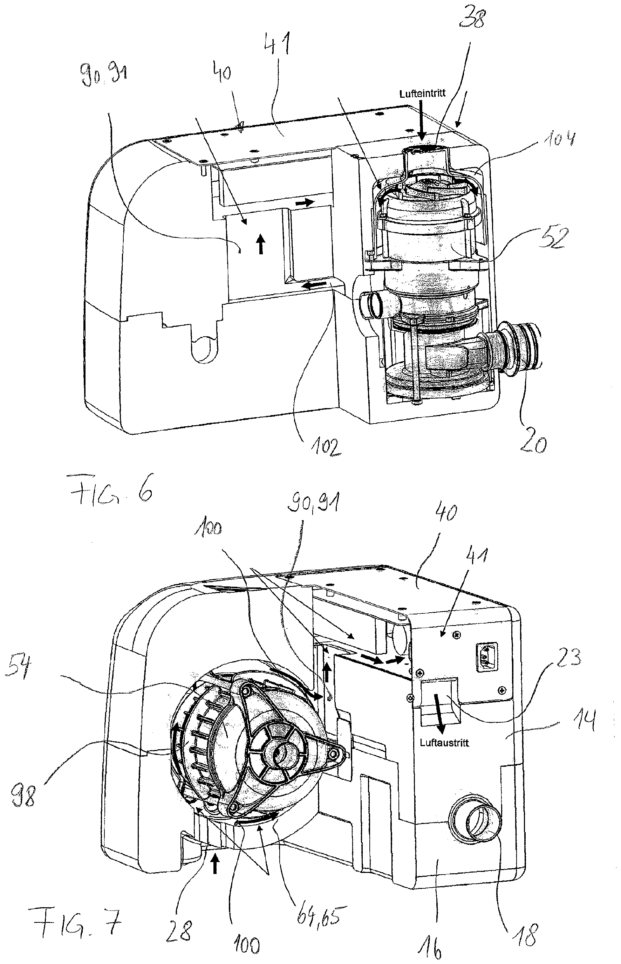

In Fig. 7 ist dieser freie Raum 100, der diesen mittleren Bereich 98 der Saugeinheit 94 umgibt, in seiner Gesamtheit zu erkennen. Dieser freie Raum 100 besitzt zudem eine Verbindung nach außen über die Öffnungen 28 im Boden der unteren Gehäuseschale 16. Zudem ist in Fig. 7 gut zu erkennen, dass der freie Raum 100 in die Öffnung 90 und den Kanal 91 mündet. Der Kanal 91 öffnet sich in einen in der oberen Gehäuseschale 14 vorgesehenen freien Bereich, der das Elektronikgehäuse 40 aufnimmt und durch die Abdeckplatte 41 nach oben und nach vorne zur Seite 22 abgedichtet ist. Lediglich über die Öffnung 23 besteht eine Verbindung nach außen.

Durch diese spezielle Ausgestaltung der beiden Gehäuseschalen 14, 16 lässt sich ein Luftstrom realisieren, der durch die Öffnungen 28 im Boden der unteren Gehäuseschale 16 angesaugt wird, im freien Raum 100 an den Kühlrippen des mittleren Bereichs 98 der Saugeinheit 54 vorbeifließt, in den Kanal 91, dann in den von der Abdeckplatte 41 begrenzten Raum gelangt und schließlich über die Öffnung 23 nach außen strömt. Dieser Luftstrom dient einerseits der Kühlung der Saugeinheit 54 und andererseits der Kühlung elektrischer Komponenten im Elektronikgehäuse 40. Die verschiedenen Flächen der beiden Gehäuseschalen 14, 16 bilden folglich Luftführungskanäle aus, ohne dass es hierfür weiterer zusätzlicher Bauteile bedarf.

In Fig. 6 ist eine weitere Schnittansicht der Saugvorrichtung 10 gezeigt, wobei die Saugeinheit 54 weggelassen und stattdessen die Separiereinheit 52 dargestellt ist. Die Schnittansicht in Fig. 6 gibt einen freien Blick auf den Kanal 91, der senkrecht nach oben in den Bereich unter der Abdeckplatte 41 des Elektronikgehäuses 40 verläuft. In diesen Kanal 91 mündet nicht nur der zuvor beschriebene freie Raum 100, der die Saugeinheit 54 umgibt, sondern auch ein Kanal 102, der waagrecht verläuft und eine Verbindung zu einem freien Raum 104 schafft. Dieser freie Raum 104 umgibt zumindest einen Teil des oberen Bereichs der Separiereinheit 52 und besitzt eine Verbindung zu der Öffnung 38 auf der Oberseite der oberen Gehäuseschale 14. Dieser freie Raum 104 ermöglicht eine Luftführung um den oberen Bereich der Separiereinheit 52 in den Kanal 102 und von dort über den Kanal 91 in den unterhalb der Abdeckplatte 41 liegenden Bereich nach außen durch die Öffnung 23. Diese Luftströmung wird von der Separiereinheit 52 erzeugt und dient hauptsächlich ihrer Kühlung. Darüber hinaus kühlt dieser Luftstrom zusätzlich auch Komponenten im Elektronikgehäuse 40.

Aus den beiden Fig. 6 und 7 ergibt sich, dass der Kanal 91 zwei Luftströmungen bündelt, die jeweils einzeln die Saugeinheit 54 bzw. die Separiereinheit 52 kühlen und gemeinsam zudem auch elektronische Komponenten im Elektronikgehäuse 40 kühlen. Die Ausgestaltung dieser Luftführungselemente erfolgt alleine durch entsprechende Ausgestaltung der beiden Gehäuseschalen 14, 16, so dass separate Bauteile, wie Schlauchleitungen etc., nicht erforderlich sind.

Insgesamt zeigt sich, dass die beiden Gehäuseschalen 14, 16 eine Vielzahl von funktionalen Komponenten ausbilden, ohne dass hierfür weitere Bauteile erforderlich wären. Zu diesen funktionalen Komponenten zählen beispielsweise die Luftführungskanäle, die beispielsweise die Luftführung um die Saugeinheit 54 und die Separiereinheit 52 und das Elektronikgehäuse 40 ermöglichen. Auch die Standfüße, Halteelemente für Leitungen, Anschlüsse, Motoren etc. oder auch die beschriebene Randabstufung als Abdichtung können als funktionale Komponenten bezeichnet werden. Darüber hinaus wird durch die entsprechende Auswahl eines schäumbaren bzw. expandierbaren Materials eine Schalldämmung und eine Schwingungsentkopplung erzielt, so dass weitere funktionale Komponenten über die Gehäuseschalen 14, 16 bereitgestellt werden. So sind beispielsweise Gummipuffer oder Ähnliches für die Schwingungsentkopplung nicht mehr erforderlich.

Ferner stellen die beiden Gehäuseschalen 14, 16 Abstützflächen bereit, die die innerhalb des Gehäuses 12 vorgesehenen Baugruppen, wie beispielsweise Separiereinheit, Saugeinheit, Elektronikgehäuse, oder beispielsweise Ventile, Leitungen etc., einerseits abstützen und andererseits innerhalb des Gehäuses fixieren.

Durch diese Maßnahmen lässt sich eine sehr kompakte, leichte Saugvorrichtung schaffen, die sehr gut schallgedämmt und schwingungsentkoppelt ist. Darüber hinaus ist die Fertigung bzw. Montage dieser Saugvorrichtung 10 deutlich vereinfacht gegenüber bisherigen Lösungen, da die Baugruppen in die Gehäuseschalen 14, 16 eingelegt werden müssen, ohne viele Schraubarbeiten vornehmen zu müssen.

## Patentansprüche

1. Saugvorrichtung für dentale, medizinische oder industrielle Zwecke mit
- einer Sauganlage, und
- einem Gehäuse (12), in das die Sauganlage (50) aufgenommen ist,
wobei
das Gehäuse (12) aus zumindest zwei zusammensetzbaren und aus einem geschäumten Material gefertigten Gehäuseschalen (14, 16) aufgebaut ist, zumindest eine der Gehäuseschalen (14, 16) Ausnehmungen (60, 64, 70, 72, 76, 78), die an Komponenten der Sauganlage (50) derart angepasst sind, dass diese in die Ausnehmungen (60, 64, 70, 72, 76, 78) eingelegt werden können, und Abstützflächen aufweist, welche die Komponenten der Sauganlage (50) im Gehäuse (12) abstützen, um die Komponenten in einer festen Position zueinander zu halten, und dass
zumindest eine der Gehäuseschalen (14, 16) zumindest eine funktionale Komponente der Sauganlage (50) ausbildet.

2. Saugvorrichtung nach Anspruch 1, **dadurch gekennzeichnet, dass** die Sauganlage (50) zumindest eine Saugeinheit (54) und/oder zumindest eine Separiereinheit (52) und/oder zumindest eine Abscheidereinheit umfasst.

3. Saugvorrichtung nach Anspruch 1 oder 2, **dadurch gekennzeichnet, dass** das geschäumte Material ein Kunststoff ist.

4. Saugvorrichtung nach Anspruch 1, 2 oder 3, **dadurch gekennzeichnet, dass** die Gehäuseschalen (14, 16) aus einem Kunststoff expandiert sind.

5. Saugvorrichtung nach einem der Ansprüche 1 bis 4, **dadurch gekennzeichnet, dass** eine funktionale Komponente ein Luftführungskanal, ein Standfuß, ein Schalldämmungselement, ein Schwingungsentkoppelungselement und/oder ein Halteelement für Leitungen oder Anschlüsse ist.

6. Saugvorrichtung nach Anspruch 5, **dadurch gekennzeichnet, dass** mehrere Standfüße (30) an zumindest einer der Gehäuseschalen (14, 16) ausgeformt sind.

7. Saugvorrichtung nach einem der vorhergehenden Ansprüche, **dadurch gekennzeichnet, dass** in zumindest einer der Gehäuseschalen (14, 16) zumindest ein Aufnahmebereich ausgeformt ist, der zur Aufnahme von zumindest einer Saugvorrichtungs-Komponente dient.

8. Saugvorrichtung nach Anspruch 7, **dadurch gekennzeichnet, dass** eine Saugvorrichtungs-Komponente, ein elektrisches Anschlusselement, ein Schlauchanschlusselement, ein Steuerungsgehäuse, eine elektrische Leitung und/oder eine Kabelführung für Leitungen ist.

9. Saugvorrichtung nach einem der vorhergehenden Ansprüche, **dadurch gekennzeichnet, dass** in zumindest einer der Gehäuseschalen (14, 16) Abstützflächen für eine Steuerungseinheit, eine Lüftereinheit, eine Separiereinheit und/oder eine Antriebseinheit ausgeformt sind.

10. Saugvorrichtung nach einem der vorhergehenden Ansprüche, **dadurch gekennzeichnet, dass** in zumindest einer der Gehäuseschalen (14, 16) zumindest eine Öffnung ausgeformt ist, die als Lufteinlassöffnung oder Luftauslassöffnung dient.

11. Saugvorrichtung nach einem der vorhergehenden Ansprüche, **dadurch gekennzeichnet, dass** das Gehäuse (12) aus zwei Gehäuseschalen (14, 16) gebildet ist.

12. Saugvorrichtung nach einem der vorhergehenden Ansprüche, **dadurch gekennzeichnet, dass** das Gehäuse (12) die Sauganlage (50) vollständig kapselt.

13. Saugvorrichtung nach einem der Ansprüche 2 bis 12, **dadurch gekennzeichnet, dass** die Saugeinheit eine Radial-Saugmaschine ist.

14. Saugvorrichtung nach einem der vorhergehenden Ansprüche 5 bis 13, **dadurch gekennzeichnet, dass** ein Luftführungskanal um einen Bereich der Saugeinheit und/oder der Separiereinheit herum geführt ist.

15. Saugvorrichtung nach Anspruch 14, **dadurch gekennzeichnet, dass** der Luftführungskanal in den Bereich der Steuerungseinheit geführt ist.

## Claims

1. Suction machine for dental, medical or industrial purposes, having
- a suction system and
- a housing (12) that accommodates the suction system (50),
wherein
the housing (12) is constructed from at least two housing shells (14, 16) that can be assembled and are made of a foamed material,
at least one of the housing shells (14, 16) has cutouts (60, 64, 70, 72, 76, 78) which are adapted to components of suction system (50) so that these can be inserted into the cutouts (60, 64, 70, 72, 76, 78), and has support faces which support the components of the suction system (50) in the housing (12), in order to hold the components in a fixed position relative to one another,
and that
at least one of the housing shells (14, 16) forms at least one functional component of the suction system (50).

2. Suction machine according to Claim 1, **characterized in that** the suction system (50) comprises at least one suction unit (54) and/or at least one separation unit (52) and/or at least one interception unit.

3. Suction machine according to Claim 1 or 2, **characterized in that** the foamed material is a plastic.

4. Suction machine according to Claim 1, 2 or 3, **characterized in that** the housing shells (14, 16) are expanded from a plastic.

5. Suction machine according to one of Claims 1 to 4, **characterized in that** a functional component is an air duct, a foot, a sound-damping element, a vibration decoupling element and/or a holding element for lines or connections.

6. Suction machine according to Claim 5, **characterized in that** multiple feet (30) are formed on at least one of the housing shells (14, 16).

7. Suction machine according to one of the preceding claims, **characterized in that** at least one receiving region is formed in at least one of the housing shells (14, 16), this region serving to receive at least one suction machine component.

8. Suction machine according to Claim 7, **characterized in that** a suction machine component, is an electrical connection element, a hose connection element, a control housing, and electrical line and/or a cable guide for lines.

9. Suction machine according to one of the preceding claims, **characterized in that** support faces for a control unit, a fan unit, a separation unit and/or a drive unit are formed in at least one of the housing shells (14, 16).

10. Suction machine according to one of the preceding claims, **characterized in that** at least one opening is formed in at least one of the housing shells (14, 16), this opening serving as an air inlet opening or air outlet opening.

11. Suction machine according to one of the preceding claims, **characterized in that** the housing (12) is formed of two housing shells (14, 16).

12. Suction machine according to one of the preceding claims, **characterized in that** the housing (12) entirely encloses the suction system (50).

13. Suction machine according to one of Claims 2 to 12, **characterized in that** the suction unit is a radial suction device.

14. Suction machine according to one of the preceding Claims 5 to 13, **characterized in that** an air duct is guided around a region of the suction unit and/or the separation unit.

15. Suction machine according to Claim 14, **characterized in that** the air duct is guided into the region of the control unit.

## Revendications

1. Dispositif d'aspiration à usage dentaire, médical ou industriel, comprenant
- une installation d'aspiration et
- un boîtier (12) dans lequel ladite installation d'aspiration (50) est logée, sachant
que ledit boîtier (12) est structurellement organisé en au moins deux coques (14, 16) pouvant être assemblées, et fabriquées en un matériau soumis à moussage,
au moins l'une desdites coques (14, 16) du boîtier comportant des évidements (60, 64, 70, 72, 76, 78) qui sont adaptés à des composants de l'installation d'aspiration (50), de telle sorte que ces derniers puissent être insérés dans lesdits évidements (60, 64, 70, 72, 76, 78), et des surfaces d'appui qui procurent un appui auxdits composants de ladite installation d'aspiration (50), dans ledit boîtier (12), de façon à maintenir lesdits composants en un emplacement fixe les uns par rapport aux autres,
et sachant
qu'au moins l'une desdites coques (14, 16) du boîtier forme au moins un composant fonctionnel de ladite installation d'aspiration (50).

2. Dispositif d'aspiration selon la revendication 1, **caractérisé par le fait que** l'installation d'aspiration (50) inclut au moins une unité d'aspiration (54) et/ou au moins une unité de ségrégation (52) et/ou au moins une unité de séparation.

3. Dispositif d'aspiration selon la revendication 1 ou 2, **caractérisé par le fait que** le matériau soumis à moussage est une matière plastique.

4. Dispositif d'aspiration selon la revendication 1, 2 ou 3, **caractérisé par le fait que** les coques (14, 16) du boîtier sont produites par expansion d'une matière plastique.

5. Dispositif d'aspiration selon l'une des revendications 1 à 4, **caractérisé par le fait qu'**un composant fonctionnel est un canal de guidage d'air, un piètement d'assise, un élément d'insonorisation, un élément de découplage vibratoire et/ou un élément de retenue affecté à des conduits ou à des raccords.

6. Dispositif d'aspiration selon la revendication 5, **caractérisé par le fait que** plusieurs piètements d'assise (30) sont venus de moulage solidaire sur au moins l'une des coques (14, 16) du boîtier.

7. Dispositif d'aspiration selon l'une des revendications précédentes, **caractérisé par le fait qu'**au moins une zone réceptrice, venue de moulage solidaire dans au moins l'une des coques (14, 16) du boîtier, sert à recevoir au moins un composant dudit dispositif d'aspiration.

8. Dispositif d'aspiration selon la revendication 7, **caractérisé par le fait qu'**un composant dudit dispositif d'aspiration est un élément de connexion électrique, un élément de raccordement de tuyaux souples, un boîtier de commande, un conducteur électrique et/ou un guide-câble dévolu à des conducteurs.

9. Dispositif d'aspiration selon l'une des revendications précédentes, **caractérisé par le fait que** des surfaces d'appui, dédiées à une unité de commande, à une unité de ventilation, à une unité de ségrégation et/ou à une unité d'entraînement, sont ménagées par moulage solidaire dans au moins l'une des coques (14, 16) du boîtier.

10. Dispositif d'aspiration selon l'une des revendications précédentes, **caractérisé par le fait qu'**au moins un orifice, servant d'orifice d'admission d'air ou d'orifice de sortie d'air, est ménagé par moulage solidaire dans au moins l'une des coques (14, 16) du boîtier.

11. Dispositif d'aspiration selon l'une des revendications précédentes, **caractérisé par le fait que** le boîtier (12) est formé de deux coques (14, 16).

12. Dispositif d'aspiration selon l'une des revendications précédentes, **caractérisé par le fait que** le boîtier (12) emprisonne l'intégralité de l'installation d'aspiration (50).

13. Dispositif d'aspiration selon l'une des revendications 2 à 12, **caractérisé par le fait que** l'unité d'aspiration est une machine d'aspiration radiale.

14. Dispositif d'aspiration selon l'une des revendications précédentes 5 à 13, **caractérisé par le fait que** le tracé d'un canal de guidage d'air ceinture une région de l'unité d'aspiration et/ou de l'unité de ségrégation.

15. Dispositif d'aspiration selon la revendication 14, **caractérisé par le fait que** l'on guide le tracé du canal de guidage d'air dans la région de l'unité de commande.
